# FASCICULE DE BREVET EUROPEEN

(11) **EP 4 106 837 B1**
(45) Date de publication et mention de la délivrance du brevet: **02.09.2026**
(21) Numéro de dépôt: 21708290.8
(22) Date de dépôt: 16.02.2021
(51) Int. Cl.: A61M 1/36

(54) **MACHINE DE DIALYSE ET SON PROCEDE DE RINCAGE**
DIALYSEMASCHINE UND IHR SPÜLVERFAHREN
DIALYSIS MACHINE AND ITS RINSING METHOD

(30) Priorité: 18.02.2020 FR 2001604
(43) Date de publication de la demande: 28.12.2022
(73) Titulaire: Physidia, 49124 Saint-Barthelemy-D'Anjou (FR)
(72) Inventeur: VINCENT, Eric, 49000 ANGERS (FR); BRUNEAU, Simon, 49000 ANGERS (FR)
(74) Mandataire: Ipsilon
(86) Numéro de dépôt international: PCT/FR2021/050269
(87) Numéro de publication internationale: WO 2021/165609

(56) Documents cités:
- WO-A2-2009/055639

## Description

### DOMAINE DE L'INVENTION

La présente invention concerne de manière générale les machines de dialyse, notamment pour l'hémodialyse, ainsi que l'utilisation (fonctionnement et manipulation) de telles machines.

### ART ANTERIEUR

De manière générale, l'hémodialyse consiste à épurer le sang de substances présentes en excès, telles que l'urée, la vitamine B12, la beta-immunoglobuline, ou encore les sels minéraux.

Une machine de dialyse comprend usuellement un circuit de dialysat pour la circulation de dialysat et un circuit extracorporel auquel un patient est raccordé lors d'une séance de dialyse. La machine de dialyse comprend aussi un dialyseur qui forme une interface de connexion entre le circuit de dialysat et le circuit extracorporel. Le dialyseur comprend un boîtier muni d'une membrane qui délimite dans le boîtier un compartiment sang et un compartiment dialysat.

L'élimination des substances en excès dans le sang du patient peut s'effectuer par osmose en faisant circuler le sang d'un côté de la membrane et le dialysat de l'autre côté de la membrane. Ainsi, les substances présentes en excès dans le sang se diffusent à travers la membrane dans le dialysat. Il est aussi possible d'extraire une fraction aqueuse du sang selon une opération appelée ultrafiltration. A l'inverse d'une opération d'ultrafiltration, il est aussi possible d'introduire une quantité appropriée de dialysat dans le sang selon une opération appelée rétrofiltration.

Avant de débuter une séance de dialyse, on fait circuler du sérum physiologique dans le circuit extracorporel, qui comprend les lignes de sang artérielle et veineuse, et le compartiment sang du dialyseur, pour rincer ce circuit extracorporel.

Cette circulation du sérum physiologique permet de rincer et ainsi de nettoyer le circuit extracorporel en enlevant les différentes particules ou détritus qui pourrait résider dans ce circuit extracorporel.

On constate cependant que des bulles d'air ont tendance à s'accumuler dans la partie haute du compartiment sang du dialyseur. Ces bulles d'air augmentent le risque de coagulation du sang du fait du contact entre le sang qui circule dans ce circuit extracorporel lors de la séance de dialyse et ces bulles d'air.

En outre lorsque ces bulles d'air sont présentes au niveau de la membrane du dialyseur, l'efficacité de la surface d'échange entre le compartiment de sang et le compartiment de dialysat est diminuée.

Pour retirer ces bulles, il a été constaté que les usagers ou opérateurs peuvent avoir tendance à manipuler le dialyseur pour le retourner ou taper dessus, afin de faire remonter les bulles dans la poche de sérum physiologique.

Cependant une telle pratique n'est pas souhaitable car il y a un risque de dégradation de la machine de dialyse et éventuellement un risque de contamination.

Le document WO2009055639 A2 décrit un appareil de dialyse qui comprend un instrument de dialyse pourvu d'un dispositif de commande de pompe et d'un premier et second dispositif de commande de soupapes, et d'une cassette jetable compatible avec l'instrument de dialyse. La cassette jetable comprend une partie pompe pouvant être commandée par le dispositif de commande de pompe, et des première et seconde chambres de soupapes pouvant être commandées par les premier et second dispositifs de commande de soupapes. Les première et seconde chambres de soupapes sont en communication fluidique l'une avec l'autre.

La présente invention a pour but de proposer une nouvelle machine de dialyse et un nouveau procédé de rinçage correspondant permettant de palier tout ou partie des problèmes exposés ci-dessus.

### RESUME DE L'INVENTION

A cet effet, l'invention a pour objet une machine de dialyse permettant de traiter un liquide corporel, tel que du sang ou du plasma, ladite machine comportant :
- un dialyseur comprenant une enceinte qui inclut :
   - une première zone de passage de liquide, appelée compartiment sang, qui présente une entrée, dite entrée de liquide corporel, et une sortie, dite sortie de liquide corporel, et
   - une deuxième zone de passage de dialysat, appelée compartiment dialysat, qui présente une entrée de dialysat et une sortie de dialysat ;
   - un système de membrane entre la première zone de passage de liquide et la zone de passage de dialysat ;
- une ligne artérielle raccordée à l'entrée de liquide corporel du compartiment sang du dialyseur ; la ligne artérielle présentant une extrémité à laquelle un premier contenant, telle qu'une poche, de sérum physiologique est raccordable;
- une ligne veineuse raccordée à la sortie de liquide corporel du compartiment sang du dialyseur ; la ligne veineuse présentant une extrémité à laquelle un deuxième contenant, telle qu'une poche, est raccordable ;
- une pompe, appelée pompe à sang, configurée pour permettre de faire circuler un liquide dans la ligne artérielle dans un sens et dans l'autre ;
- un système d'amenée du dialysat comprenant :
   - une ligne d'amenée de dialysat qui est raccordée à l'entrée de la zone de passage de dialysat, et
   - un système de délivrance de dialysat raccordé à la ligne d'amenée, et auquel est raccordable une source d'alimentation en dialysat ;
- un système d'évacuation du dialysat comprenant une ligne d'évacuation de dialysat qui est raccordée à la sortie du compartiment de dialysat ;
   l'unité de pilotage étant configurée pour, à l'état raccordé, du premier contenant à la ligne artérielle, du deuxième contenant à la ligne veineuse, et de la source d'alimentation en dialysat au système de délivrance de dialysat,
   mettre en œuvre une première phase de rinçage, appelée phase de rinçage de circuit extracorporel, qui comprend une étape de commande de fonctionnement de la pompe à sang de manière à faire circuler le sérum physiologique contenu dans le premier contenant jusque dans le deuxième contenant ;
   caractérisée en ce que l'unité de pilotage est configurée pour mettre en œuvre une phase d'élimination d'air dans le dialyseur, qui comprend les étapes suivantes:
      - commander la fermeture de circulation dans la ligne veineuse ;
      - commander la fermeture de circulation dans la ligne d'évacuation du système d'évacuation du dialysat ;
      - commander le fonctionnement du système de délivrance du dialysat pour faire circuler du dialysat depuis la source d'alimentation en dialysat à travers le compartiment sang et la ligne artérielle.

Une telle conception de la machine permet de rincer le circuit extracorporel de la machine de dialyse tout en éliminant efficacement les bulles d'air susceptibles d'être bloquées dans le haut du dialyseur, et ce sans risque de dégradation et/ou de contamination de la machine de dialyse.

Par ailleurs le fait d'éliminer les bulles en faisant circuler du dialysat depuis la source d'alimentation en dialysat et dans le sens d'une remontée de la ligne artérielle jusque dans le premier contenant de sérum physiologique, permet de compenser au moins en partie la quantité de sérum physiologique écoulée du premier contenant vers le deuxième contenant de recueil au cours de la phase de rinçage.

Il est ainsi possible d'utiliser un contenant de volume réduit comparé à celui utilisé dans l'état de la technique pour réaliser l'étape de rinçage. Le premier contenant peut alors être une poche de 1L au lieu d'une poche de 2L utilisée dans l'état de la technique. En effet, dans l'état de la technique, la poche devait être de contenance suffisante pour conserver un certain volume de sérum physiologique en prévision d'une phase de restitution en fin de séance de dialyse. Avec l'invention, le dialysat qui est collecté dans la poche de sérum physiologique, en se mélangeant avec le sérum physiologique qui reste à l'issue de la phase de rinçage, peut être utilisé dans la phase de restitution. Il est à noter que la composition du sérum physiologique est proche de celle du dialysat.

La machine peut aussi comporter une ou plusieurs des caractéristiques suivantes prises dans toute combinaison techniquement admissible.

Selon une caractéristique avantageuse, l'entrée de liquide corporel du dialyseur est située au-dessus de la sortie de liquide corporel.

Selon une caractéristique avantageuse, la ligne veineuse est munie d'un système de pince, commandable par l'unité de pilotage, pour permettre de fermer ou d'ouvrir la circulation à l'intérieur de la ligne veineuse.

Selon une caractéristique avantageuse, pour l'exécution de la phase d'élimination d'air, l'unité de pilotage est configurée pour commander le fonctionnement de la pompe à sang, dans le sens inverse du sens de fonctionnement utilisé lors de la phase de rinçage, parallèlement à ladite étape de commande de fonctionnement du système de délivrance du dialysat.

Selon une caractéristique avantageuse, la machine de dialyse comprenant un capteur de pression agencé pour mesurer la pression dans le circuit de circulation de dialysat formé entre la pompe et le système de délivrance de dialysat, l'unité de pilotage est configurée pour piloter la vitesse de fonctionnement de la pompe en fonction de la pression mesurée de manière à maintenir ladite pression dans une plage de valeur prédéfinie.

Selon une caractéristique avantageuse, l'unité de pilotage est configurée pour, dans la phase de rinçage et/ou d'élimination d'air, faire varier la vitesse de fonctionnement de la pompe afin de générer des à-coups.

Selon une caractéristique avantageuse, ledit système de délivrance comprend au moins une poche souple, appelée poche ventricule, destinée à contenir du dialysat, et des moyens de mise sous pression de la poche ventricule.

Selon une caractéristique avantageuse, l'unité de pilotage est configurée pour déterminer le volume de sérum physiologique déplacé par la pompe à sang jusque dans le deuxième contenant, et pour arrêter de faire fonctionner ladite pompe à sang lorsque le volume de sérum physiologique déplacé a atteint une valeur prédéfinie, par exemple comprise entre 600 et 1800 mL.

L'invention concerne également un procédé de rinçage et d'élimination d'air pour une machine de dialyse, ladite machine de dialyse comportant :
- un dialyseur comprenant une enceinte qui inclut :
   - une première zone de passage de liquide appelée compartiment sang, qui présente une entrée, dite entrée de liquide corporel, et une sortie, dite sortie de liquide corporel, et
   - une deuxième zone de passage de dialysat, appelée compartiment dialysat, qui présente une entrée de dialysat et une sortie de dialysat ;
   - un système de membrane entre la première zone de passage de liquide et la zone de passage de dialysat ;
   - une ligne artérielle raccordée à l'entrée de liquide corporel du compartiment sang du dialyseur ; la ligne artérielle présentant une extrémité à laquelle un premier contenant, telle qu'une poche, de sérum physiologique est raccordable ;
   - une ligne veineuse raccordée à la sortie de liquide corporel du compartiment sang du dialyseur ; la ligne veineuse présentant une extrémité à laquelle un deuxième contenant, telle qu'une poche, est raccordable;
   - une pompe, appelée pompe à sang, configurée pour permettre de faire circuler un liquide dans la ligne artérielle dans un sens et dans l'autre ;
   - un système d'amenée du dialysat comprenant :
      - une ligne d'amenée de dialysat qui est raccordée à l'entrée de la zone de passage de dialysat, et
   - un système de délivrance de dialysat raccordé à la ligne d'amenée, et auquel est raccordable une source d'alimentation en dialysat ;
   - un système d'évacuation du dialysat comprenant une ligne d'évacuation de dialysat qui est raccordée à la sortie du compartiment de dialysat ;
      ledit procédé comprenant une première phase de rinçage, appelée phase de rinçage de circuit extracorporel, qui comprend une étape de commande de fonctionnement de la pompe à sang de manière à faire circuler le sérum physiologique contenu dans le premier contenant jusque dans le deuxième contenant,
      caractérisé en ce que ledit procédé comprend aussi une phase d'élimination d'air dans le dialyseur, qui comprend les étapes suivantes :
         - fermeture de circulation dans la ligne veineuse ;
         - fermeture de circulation dans la ligne d'évacuation du système d'évacuation du dialysat ;
         - fonctionnement du système de délivrance du dialysat pour faire circuler du dialysat depuis la source d'alimentation en dialysat à travers le compartiment sang et la ligne artérielle.

Selon une caractéristique avantageuse, la phase d'élimination d'air comprend le fonctionnement de la pompe à sang, dans le sens inverse du sens de fonctionnement utilisé lors de la phase de rinçage, parallèlement à ladite étape de fonctionnement du système de délivrance du dialysat.

L'invention concerne également un produit de programme informatique non transitoire comprenant des instructions de code de programme pour l'exécution des étapes d'un procédé tel que décrit ci-dessus, lorsque ledit programme est exécuté par un processeur d'une unité de pilotage d'une machine de dialyse telle que décrite ci-dessus.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
- [Fig. 1] la Figure 1 est une vue schématique du circuit extracorporel d'une machine de dialyse conformément à un mode de réalisation de l'invention, la machine de dialyse étant configurée pour une phase de rinçage du circuit extracorporel ;
- [Fig. 2] la Figure 2 est une vue schématique du circuit extracorporel d'une machine de dialyse conformément à un mode de réalisation de l'invention, la machine de dialyse étant configurée pour une phase d'élimination d'air (par rinçage en sens inverse vis-à-vis de l'entrée du dialyseur raccordée à la ligne artérielle) ;
- [Fig. 3] la Figure 3 est une vue schématique du dialyseur d'une machine de dialyse conformément à un mode de réalisation de l'invention, au cours d'une phase d'élimination d'air ;
- [Fig. 4] la Figure 4 est une vue du circuit extracorporel et du circuit de dialysat d'une machine de dialyse selon un mode de réalisation de l'invention et selon une configuration de fonctionnement correspondant à une phase de rinçage ;
- [Fig. 5] la Figure 5 est une vue du circuit extracorporel et du circuit de dialysat d'une machine de dialyse selon un mode de réalisation de l'invention et selon une configuration de fonctionnement correspondant à une phase d'élimination d'air ;
- [Fig. 6] la Figure 6 est un logigramme montrant des étapes d'un procédé de rinçage selon un mode de réalisation de l'invention.

### DESCRIPTION DETAILLEE

L'invention est décrite plus complètement ci-après avec référence aux dessins joints, sur lesquels des modes de réalisation de l'invention sont montrés. Sur les dessins, la taille et les tailles relatives des éléments peuvent être exagérées à des fins de clarté. Des numéros similaires font référence à des éléments similaires sur tous les dessins. Cependant, l'invention peut être mise en œuvre sous de nombreuses formes différentes et ne devrait pas être interprétée comme étant limitée aux modes de réalisation exposés ici. Les modes de réalisation qui suivent sont examinés, par souci de simplification, en relation avec la terminologie et la structure d'une machine de dialyse et d'un procédé de rinçage et d'élimination d'air correspondant.

Une référence dans toute la description à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation. En particulier, l'ordre des étapes de procédé peut être modifié lorsque cela reste techniquement admissible.

### • Aspects généraux

En référence aux figures et comme rappelé ci-dessus, l'invention concerne une machine de dialyse et un procédé de rinçage permettant de rincer efficacement le circuit extracorporel de la machine de dialyse avant une séance de dialyse, en limitant la présence de bulles dans le circuit extracorporel et en particulier dans le compartiment sang du dialyseur.

La machine de dialyse permet de traiter un liquide corporel, tel que du sang ou du plasma. Par liquide corporel, on entend un liquide du type de ceux présents dans le corps humain ou animal, tel que du sang ou du plasma. Dans la suite de la description, le liquide corporel considéré est du sang mais bien entendu pourrait être un autre liquide corporel tel que du plasma.

Selon un mode de réalisation la machine de dialyse peut comprendre les mêmes éléments que ceux décrits dans la demande internationale numéro WO2013050689A1 ou WO2019150058A2. La machine de dialyse selon l'invention se distingue cependant de celles décrites dans lesdites demandes internationale WO2013050689A1 et WO2019150058A2 par un procédé de rinçage particulier mis en œuvre à l'aide d'instructions informatiques et/ou électroniques exécutées par une unité de pilotage qui est présentée ci-après.

### • Dialyseur

Ladite machine comporte un dialyseur 100. Le dialyseur est encore usuellement appelé filtre dialyseur. Le dialyseur 100 se présente sous la forme d'une enceinte qui inclut une zone de passage du liquide corporel 12, appelée compartiment sang, qui présente une entrée 201 de liquide corporel et une sortie 202 de liquide corporel. L'enceinte inclut aussi une zone de passage de dialysat 14 qui présente une entrée de dialysat 401 et une sortie de dialysat 402.

Il est à noter que dans le procédé de rinçage présenté ci-après, le liquide qui circule à travers le compartiment sang 12 n'est pas un liquide corporel mais du sérum physiologique ou du dialysat. Pour autant les dénominations usuelles de « compartiment sang » et entrée/sortie de « liquide corporel » ou « pompe à sang », qui font référence à une utilisation lors d'une séance de dialyse, sont conservées.

Le dialysat est un liquide bien connu de l'homme du métier dont la composition est proche du sérum physiologique. Le dialysat comprend de l'eau d'une qualité suffisante sur le plan minéral et sur le plan bactériologique. Cette eau est additionnée à un concentré, sous forme de poudre de sels minéraux ou sous forme de concentré liquide, pour fabriquer le dialysat requis.

Usuellement le sérum physiologique est composé d'un mélange d'eau et de chlorure de sodium. Le dialysat est généralement composé de sérum physiologique auquel sont ajoutés du glucose et des ions tels que potassium, magnésium calcium et éventuellement lactate et/ou bicarbonate selon les besoins du patient.

Selon un aspect préférentiel, le dialysat utilisé est du dialysat stérile apyrogène. Le dialysat a été stérilisé par exemple dans un autoclave. Ce dialysat peut être réalisé à partir d'eau purifiée et de sel.

La circulation de dialysat passe par le compartiment du dialysat 14, généralement à contre-sens de la circulation sanguine dans l'autre compartiment sang 12. Le dialysat circule généralement en circuit ouvert. On peut aussi prévoir une boucle de régénération partielle de dialysat propre à partir du dialysat chargé des toxines.

### • Membrane

L'enceinte inclut aussi un système de membrane 3 pour permettre un échange d'éléments (substances, molécules) entre le liquide corporel présent dans la zone de passage de liquide corporel 12 et le dialysat présent dans la zone de passage de dialysat 14.

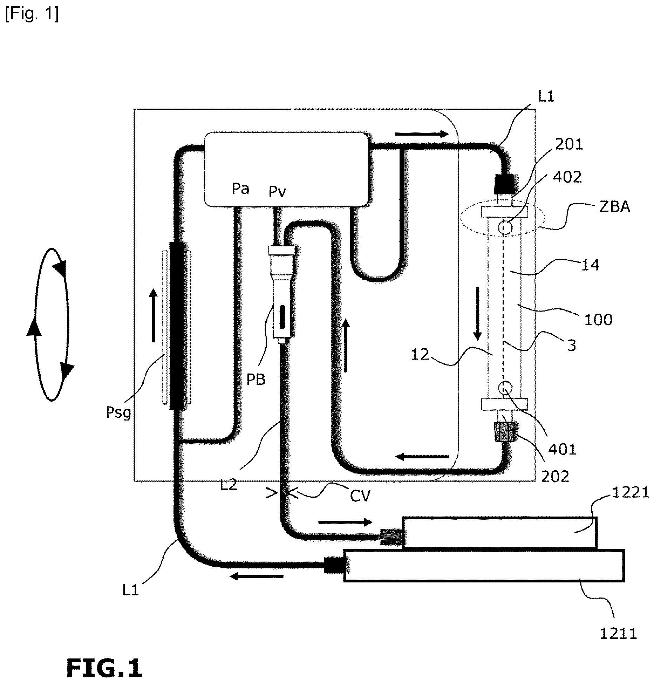

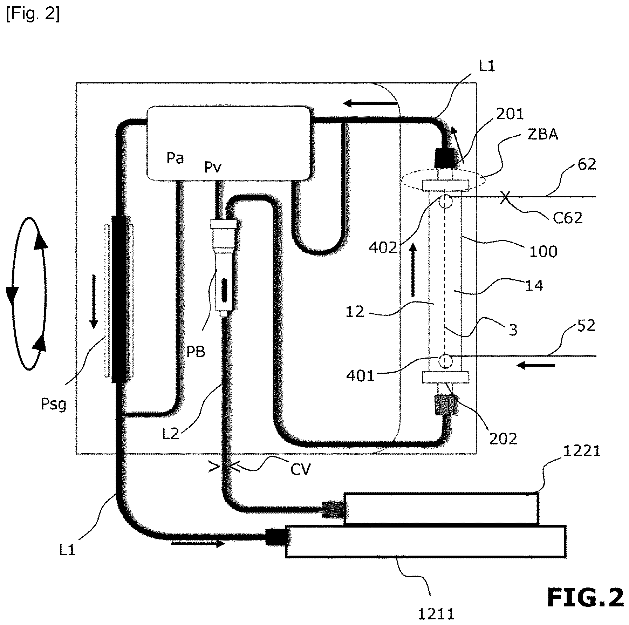

Comme illustré aux Figures 1 et 2, d'un point de vue fonctionnel, le dialyseur 100 peut être schématisé sous la forme d'une enceinte logeant une membrane 3 de dialyse qui sépare l'enceinte en un compartiment sang, correspondant à la zone de passage du liquide corporel 12 et en un compartiment dialysat, correspondant à la zone de passage de dialysat 14.

Le système de membrane 3 de dialyse, semi-perméable, est conçu de manière à laisser passer une fraction du volume sanguin lorsque la différence entre la pression locale dans le compartiment sang 12 et la pression locale dans le compartiment dialysat 14 est supérieure à une valeur donnée. Cette différence de pression est appelée pression transmembranaire. En particulier, le système de membrane est conçu de telle sorte que seules les molécules les plus petites peuvent passer à travers les trous ou pores du système de membrane, à savoir l'eau, les sels minéraux et les molécules de petit à moyen poids moléculaire.

Plus précisément, le système de membrane 3 peut comprendre une membrane conçue de telle sorte que, lorsque la différence entre la pression qui s'exerce sur au moins une portion de la membrane du côté du compartiment sang 12 et la pression qui s'exerce sur ladite portion de la membrane du côté du compartiment de dialysat 14, est supérieure à une valeur seuil donnée, ladite portion de la membrane laisse passer une fraction aqueuse du sang dans le compartiment de dialysat 14, selon un phénomène convectif appelé ultrafiltration. Inversement, ladite membrane 3 est conçue de telle sorte que, lorsque la différence entre la pression, qui s'exerce sur ladite portion de la membrane 3 du côté du compartiment de dialysat 14, et la pression, qui s'exerce sur ladite portion de la membrane du côté du compartiment sang 12, est supérieure à une valeur seuil donnée, ladite portion de la membrane laisse passer le dialysat dans le compartiment sang 12, selon un phénomène appelé rétrofiltration.

Le système de membrane de dialyse peut être réalisé sous forme de fibres capillaires, le sang circulant à l'intérieur des fibres, le dialysat à l'extérieur.

L'entrée 201 de sang est raccordée à une ligne artérielle L1 dont une extrémité est, pour procéder à une séance de dialyse, raccordée à une fistule du patient, afin de pouvoir extraire le sang du corps du patient à traiter. En outre, la sortie 202 de sang est raccordée à une ligne veineuse L2, dont une extrémité est raccordée à une veine du patient pour réintroduire le sang dans le corps du patient après traitement. On entend par "ligne" une tubulure comprenant éventuellement plusieurs branches ou portions et à travers laquelle un liquide peut circuler. Selon un aspect particulier ces lignes peuvent être liées l'une à l'autre de sorte que l'on ne peut retirer l'une sans l'autre.

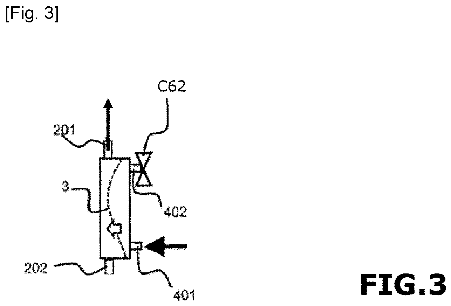

Dans l'exemple illustré aux Figures 1 à 4, la ligne artérielle L1 est aussi munie d'une pompe à sang Psg, et d'un capteur de pression Pa, encore appelé capteur de pression artérielle. La ligne veineuse L2 comprend un capteur de pression Pv, encore appelé capteur de pression veineuse, un piège à bulle PB, et un détecteur d'air DA. Avantageusement, le capteur de pression Pv est connecté sur le piège à bulle PB. Préférentiellement, ladite ligne veineuse L2 comprend aussi un système d'injection d'anticoagulant, tel qu'une pompe à héparine PH.

La ligne artérielle L1, la ligne veineuse L2 et le compartiment sang 12 forment le circuit sanguin de la machine.

Ledit compartiment de dialysat 14 présente une entrée de dialysat 401 et une sortie de dialysat 402.

### • Circuit de dialysat

La machine comprend aussi un système d'amenée du dialysat 5 qui comporte une ligne d'amenée 52 de dialysat qui est raccordée à l'entrée 401 du compartiment de dialysat 14, et un système d'évacuation du dialysat 6 qui comporte une ligne d'évacuation 62 raccordée à la sortie 402 du compartiment de dialysat 14.

Ledit système d'amenée du dialysat 5, le compartiment de dialysat 14, et ledit système d'évacuation du dialysat 6 forment le circuit de dialysat.

La ligne d'amenée 52 de dialysat est raccordée à l'entrée 401 de la zone de passage de dialysat 14 par un raccord rapide, par exemple un raccord de type Hansen. Les raccords de type Hansen correspondent à la norme NF EN ISO 8637.

Le système d'amenée 5 comprend aussi un système de délivrance 51 de dialysat raccordé à la ligne d'amenée 52. Ledit système d'amenée du dialysat 5 permet de délivrer du dialysat dans le compartiment dialysat 14 par la ligne d'amenée 52.

Ledit système de délivrance 51 comprend au moins une poche 50 souple, appelée poche ventricule, destinée à contenir du dialysat. Ledit système 51 peut comprendre plusieurs poches comme dans l'exemple illustré aux figures.

Préférentiellement, ledit système d'amenée du dialysat 5 comprend une poche ventricule supplémentaire 50' qui est montée en dérivation de la portion de la ligne d'amenée 52 sur laquelle est raccordée ladite poche ventricule 50.

Dans la suite de la description, il est fait référence à un système 51 comprenant plusieurs poches 50, 50', mais on comprend que cette description s'applique aussi au cas d'un système de délivrance 51 comprenant une seule poche. Chaque poche ventricule du système 51 est destinée à contenir du dialysat et est raccordée à ladite ligne d'amenée 52.

Chaque poche ventricule 50, 50' loge dans une enceinte 500, 500' sensiblement étanche qui peut être mise sous pression, et éventuellement en dépression. Chaque poche ventricule 50, 50' peut ainsi être mise sous pression par des moyens de mise sous pression 70 et, éventuellement, en dépression par un générateur de vide 80.

Lesdits moyens de mise sous pression 70 comprennent un dispositif d'injection de gaz sous pression, tel qu'un compresseur d'air ou une bouteille de gaz comprimé, apte à injecter du gaz sous pression dans l'enceinte 500, 500' étanche dans laquelle loge la poche ventricule 50, 50'. Ladite enceinte présente une sortie dont l'ouverture est contrôlable par une électrovanne pour réduire la pression dans l'enceinte.

L'application d'une pression sur la poche ventricule 50 ou 50' et la régulation de cette pression appliquée, à l'aide de la différence de pression mesurée aux bornes du tube à perte de charge 520, permet de maintenir pendant un temps donné un débit fixe de dialysat dans le compartiment dialysat de manière fiable et précise, et ainsi de maîtriser des opérations de rétrofiltration. L'application et la régulation de la pression appliquée sur la poche ventricule 50 ou 50' permet aussi, en ajustant la pression appliquée à la poche de décharge 60 ou 60', de maintenir pendant un temps donné une pression moyenne donnée dans le compartiment dialysat, et ainsi de maitriser des opérations d'ultrafiltration.

Les deux poches ventricules 50, 50' sont aptes à être mises sous pression/dépression indépendamment l'une de l'autre.

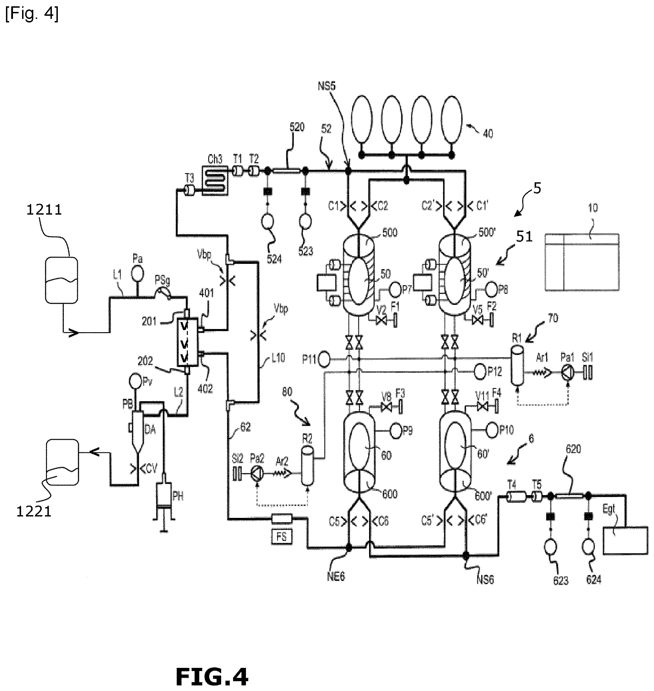

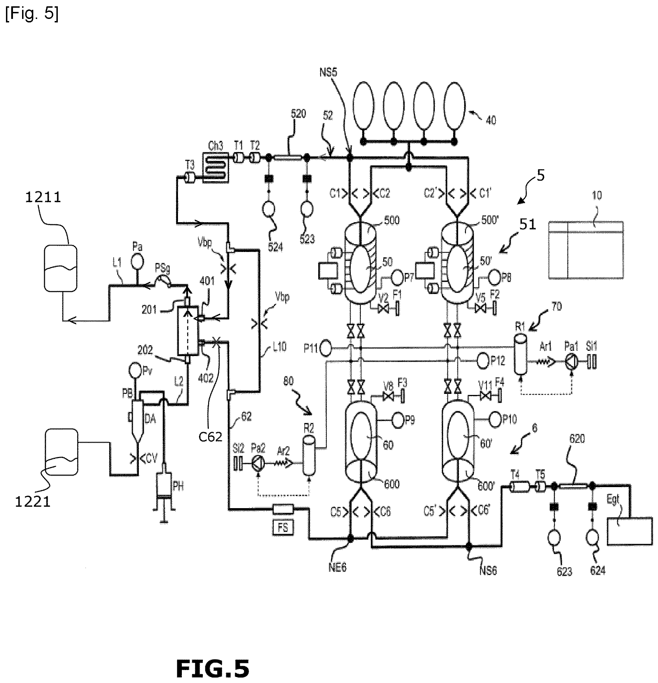

Comme illustré à la Figure 4 ou 5, chaque poche ventricule 50, 50' est aussi munie d'un organe aval de fermeture/ouverture C1, C1' de la ligne d'amenée qui, à l'état ouvert, autorise l'écoulement de dialysat depuis la poche 50, 50' correspondante vers l'entrée 401 du compartiment dialysat, sous l'effet d'une pression appliquée sur ladite poche ventricule par les moyens 70 correspondants, et à l'état fermé, empêche ledit écoulement de dialysat.

Chaque organe aval de fermeture/ouverture C1, C1' de la ligne d'amenée est situé entre la poche correspondante 50, 50' et le nœud de sortie NS5 de raccordement des deux poches 50, 50'. Chaque poche ventricule 50, 50' est aussi munie d'un organe amont de fermeture/ouverture C2, C2' qui permet d'autoriser ou non l'alimentation de ladite poche ventricule 50, 50' par une source d'alimentation 40. Selon un autre mode de réalisation, par exemple comme décrit dans la demande WO2019150058A2, il est possible d'utiliser un système de raccordement de plusieurs sources d'alimentation, la ou les sources supplémentaires pouvant servir de réserve.

Chaque portion de la ligne d'amenée 52 de dialysat au niveau de laquelle est situé un organe amont C2, C2' ou aval C1, C1' de fermeture/ouverture, est souple. Chacun desdits organes C2, C2', C1, C1' de fermeture/ouverture est formé par une pince commandable pour, d'une part, pincer de l'extérieur la paroi de ladite portion souple de la ligne d'amenée 52 de manière à fermer cette portion, et, d'autre part, pour laisser ouverte ladite portion souple de ladite ligne d'amenée 52.

La présence des deux poches ventricules et du système de pinçage alternatif formé par les organes C1 à C2' permet de charger successivement et alternativement les poches ventricules à partir de la source d'alimentation 40. Ceci permet d'alimenter en continu la ligne d'amenée du dialysat à partir de l'une des poches ventricules.

La mise en dépression de l'enceinte d'une poche ventricule 50 ou 50' à l'aide du générateur de vide 80 permet, à l'état ouvert de l'organe amont d'ouverture/fermeture C2 ou C2', et à l'état fermé de l'organe aval d'ouverture/fermeture correspondant C1 ou C1', d'aspirer le dialysat contenu dans la source d'alimentation 40 ou 40' pour remplir ladite poche ventricule 50 ou 50'.

Pour commander l'écoulement de dialysat dans la ligne d'amenée 52, l'unité de pilotage 10 commande l'ouverture de l'organe aval C1 ou C1' de l'une des poches ventricules 50 ou 50' et la fermeture de l'organe amont C2 ou C2' correspondant. L'utilisation de deux poches ventricules montées en parallèle permet d'en recharger une pendant que l'autre est en cours d'utilisation.

La ligne 52 d'amenée de dialysat comporte une restriction de passage 520 pour créer une perte de charge et permettre de réguler la pression appliquée à la poche ventricule 50 ou 50' en fonction de la différence de pression mesurée aux bornes de ladite restriction. En particulier, ladite ligne 52 est munie de moyens de mesure de la différence de pression entre l'entrée et la sortie de ladite restriction de passage 520. Cette restriction 520 peut être formée au moins en partie d'un tube calibré de section constante qui forme un étranglement sur une longueur prédéterminée. Ladite restriction de passage 520 est située entre les poches 50, 50' et l'entrée 401 du compartiment dialysat 14.

La connexion NS5 du système de délivrance 51 à ladite ligne d'amenée 52 est située en amont de la restriction de passage 520.

Lesdits moyens de mise sous pression maintiennent avantageusement une pression sur la poche ventricule 50 ou 50' qui est suffisante pour provoquer l'écoulement du dialysat. La ligne d'amenée est calibrée pour que, pour une pression déterminée appliquée à la poche ventricule 50 ou 50', le débit de dialysat dans ladite ligne ait une valeur sensiblement constante.

Un tel système formé d'un tube à perte de charge et de moyens de mesure de la différence de pression aux bornes de la restriction, permet notamment de déterminer le débit de dialysat qui circule dans ladite ligne d'amenée et qui entre dans le compartiment dialysat. Grâce au débit de dialysat ainsi mesuré, l'unité de pilotage 10 peut réguler la pression appliquée à la poche ventricule 50 ou 50' pour obtenir le débit de dialysat souhaité entrant dans le compartiment dialysat et maintenir précisément et de manière fiable ce débit à une valeur donnée pendant un temps donné.

Lesdits moyens de mesure de la différence de pression aux bornes de la restriction 520 de la ligne d'amenée 52 peuvent comprendre deux orifices de prise de pression ménagés, dans la paroi périphérique de ladite ligne 52, l'un en amont et l'autre en aval de ladite restriction 520. Chaque orifice de prise de pression est associé à un capteur de pression 523, 524. Chaque capteur de pression 523, 524 est agencé par rapport à l'orifice de prise de pression correspondant de manière à mesurer la pression dans la ligne d'amenée 52 au niveau dudit orifice de prise de pression tout en étant écarté dudit orifice pour ne pas être en contact avec le dialysat circulant dans la ligne 52, 62.

Les capteurs de pression 523, 524 peuvent être configurés comme décrits dans la demande internationale WO2013050689.

On peut prévoir que la mesure de pression dans la ligne artérielle L1 et la ligne veineuse L2 soit réalisée de la même manière que pour la prise de pression aux bornes de la restriction 520. On peut ainsi prévoir que le capteur de pression Pa et le capteur de pression Pv soient fixés dans une partie du bâti à écartement d'un orifice de pression ménagé dans la ligne L1, L2 correspondante. Selon un aspect particulier, les capteurs de pression Pa et Pv mesurent la pression dans la ligne L1, L2 correspondante sans risque de contamination.

### • Système d'évacuation du dialysat 6

Le système d'évacuation du dialysat 6 comprend une ligne d'évacuation 62 de dialysat qui est raccordée à la sortie 402 du compartiment de dialysat 14, de préférence par un raccord rapide, par exemple un raccord de type Hansen.

Ladite machine comprend un système, de préférence de type pince, pour l'ouverture/fermeture de la ligne 62 d'évacuation de dialysat. Le système d'ouverture/fermeture autorise, à l'état d'ouverture, la circulation dans ladite ligne d'évacuation 62 du dialysat présent en sortie 402 du compartiment de dialysat 14 pour remplir l'une des poches de décharge 60 ou 60', et empêche, à l'état de fermeture, la circulation dans ladite ligne d'évacuation 62 du dialysat de sorte que, pour une pression suffisante dans le compartiment dialysat, ledit dialysat passe à travers la membrane 3 dans le compartiment sang.

Dans l'exemple illustré aux Figures 4 et 5, ledit système d'évacuation du dialysat 6 comprend au moins une poche de décharge 60, de préférence souple, présentant une entrée et une sortie raccordées à la ligne d'évacuation de dialysat 62. Ledit système d'ouverture/fermeture comprend un organe amont d'ouverture/fermeture C5 situé entre la sortie 402 du compartiment dialysat 14 et ladite poche de décharge 60. Comme détaillé ci-après et représenté à la Figure 5, il est aussi prévu un organe C62 de fermeture/ouverture sur la ligne 62.

Avantageusement, ledit système d'évacuation du dialysat 6 comprend au moins une autre poche de décharge 60' montée en dérivation de ladite poche de décharge 60. Ainsi la poche de décharge 60 et la poche de décharge 60' sont situées sur deux branches de la ligne d'évacuation 62. Autrement dit, en partant de la sortie du compartiment de dialysat 14, la ligne d'évacuation 62 se sépare, en amont des poches de décharge 60, 60', en deux branches parallèle munies l'une de la poche de décharge 60 et l'autre de la poche de décharge 60'. Ces deux branches se raccordent l'une à l'autre en aval des poches 60, 60' et en amont de la restriction de section 620 comme détaillé ci-après.

Ledit système d'ouverture/fermeture comprend un autre organe amont d'ouverture/fermeture C5' situé entre ladite autre poche de décharge 60' et le nœud d'entrée NE6 de raccordement des poches de décharge 60, 60'.

De manière similaire aux organes amont C5, C5', chaque poche de décharge 60, 60' est munie d'un organe aval C6, C6' de fermeture/ouverture situé entre la poche de décharge 60, 60' correspondante et le nœud de sortie NS6 de raccordement des poches de décharge 60, 60'.

Chaque portion de la ligne d'évacuation de dialysat 62 au niveau de laquelle est situé un organe amont C5, C5' ou aval C6, C6' de fermeture/ouverture est souple. Chacun desdits organes amont C5, C5' ou aval C6, C6' de fermeture/ouverture de la ligne d'évacuation de dialysat 62 est formé par une pince commandable pour, d'une part, pincer de l'extérieur la paroi de ladite portion souple de la ligne d'évacuation 62 de manière à fermer cette portion, et, d'autre part, pour laisser ouverte ladite portion souple de ladite ligne d'évacuation 62.

Un tel ensemble d'organes amont C5, C5' et aval C6, C6' de fermeture/ouverture associés aux poches de décharge 60, 60' permet à l'état ouvert de l'organe amont C5, respectivement C5', et fermé de l'organe aval C6, respectivement C6', de remplir la poche de décharge 60, respectivement 60', et, à l'état fermé de l'organe amont C5, respectivement C5', et ouvert de l'organe aval C6, respectivement C6', de vider ladite poche de décharge 60, respectivement 60'.

Dans le cas où le système d'évacuation 6 est formé par une ou plusieurs poches de décharge 60, 60', on considère que la ligne d'évacuation 62 est ouverte lorsque le dialysat peut circuler à travers ladite ligne pour remplir ladite ou l'une desdites poches.

Les moyens d'évacuation du dialysat et du liquide présents dans la poche de décharge 60 ou 60' peuvent être formés par la gravité si la configuration de la poche de décharge s'y prête et/ou par des moyens de mise sous pression 70, de préférence communs à ceux qui servent à la mise sous pression des poches ventricules 50, 50'. Dans ce dernier cas, ladite ou chaque poche 60, 60' est logée dans une enceinte 600, 600' sensiblement étanche apte à être mise sous pression par lesdits moyens de mise sous pression 70. Ladite enceinte présente une sortie 601 dont l'ouverture est contrôlable par une électrovanne pour réduire la pression dans l'enceinte.

On peut aussi prévoir que chaque enceinte 600, 600' qui loge une poche de décharge 60, 60' soit raccordée à un générateur de vide 80. Le générateur de vide 80 et/ou les moyens de mise sous pression 70 permettent d'appliquer une pression ou une dépression à la poche de décharge 60 ou 60', de manière à ajuster la pression moyenne dans le compartiment de dialysat 14, par exemple lors d'une phase d'ultrafiltration. Dans ce cas, l'organe aval C6 ou C6' de la poche de décharge 60 ou 60' qui est utilisée pour ajuster la pression moyenne dans le compartiment de dialysat 14, est fermé tandis que l'organe amont C5 ou C5' correspondant est ouvert.

Ledit générateur de vide 80 peut être commun pour la mise en dépression des enceintes du système d'amenée 5 de dialysat et la mise en dépression des enceintes du système d'évacuation 6.

Ladite machine comprend des moyens de détermination de la quantité de dialysat et de liquide récupérée dans la poche de décharge 60 ou 60' qui se vide.

Lesdits moyens de détermination de la quantité de dialysat et de liquide récupérée dans la ou les poches de décharge 60, 60' comprennent une restriction de passage 620, ménagée dans la ligne 62 d'évacuation de dialysat et située en aval desdites poches de décharge 60, 60', et des moyens de mesure de la différence de pression aux bornes de ladite restriction de passage 620.

Lesdits moyens de mesure de la différence de pression aux bornes de la restriction 620 de la ligne d'évacuation 62 peuvent être similaires à ceux associés à la restriction 520 de la ligne d'amenée 52.

Ainsi, lesdits moyens de mesure de la différence de pression aux bornes de la restriction 620 de la ligne d'évacuation 62 peuvent comprendre deux orifices de prise de pression ménagés, dans la paroi périphérique de ladite ligne 62, l'un en amont et l'autre en aval de ladite restriction 620. Selon un aspect particulier, chaque orifice de prise de pression est associé à un capteur de pression agencé de manière à mesurer la pression dans la ligne 62 au niveau dudit orifice tout en étant écarté dudit orifice pour ne pas être en contact avec le dialysat circulant dans la ligne 62.

De manière similaire aux moyens de mesure de la différence de pression aux bornes de la restriction 520, un filtre perméable à l'air et imperméable aux agents infectieux et aux liquides, peut être interposé entre chaque capteur de pression 623, 624 et l'orifice de prise de pression correspondant. En particulier, chaque capteur de pression 623, 624 est monté dans une cavité du bâti de la machine à l'aide d'une pièce support creuse qui est destinée à être couplée au conduit 621, 622 relié à l'orifice de prise de pression correspondant.

Les capteurs de pression 623, 624 associés à ladite restriction 620 permettent de déterminer la différence de pression aux bornes de la restriction 620 et ainsi le débit de dialysat et de liquide évacué de la poche de décharge 60 ou 60', ce qui permet de déterminer, en mesurant le temps d'écoulement correspondant, la quantité de dialysat et de liquide récupérée dans ladite poche de décharge 60 ou 60'.

### • Unité de pilotage

Comme évoqué précédemment, ladite machine comprend aussi une unité de pilotage 10, telle qu'un automate programmable. L'unité de pilotage peut être configurée pour exécuter des étapes de dialyse, telles que décrites dans les demandes internationales WO2013050689A1 ou WO2019150058A2.

Une séance de dialyse comprend, outre des phases d'amorçage et de restitution, une phase de dialyse qui peut comprendre une pluralité d'étapes d'ultrafiltration en alternance avec des étapes de rétrofiltration. Un exemple de fonctionnement de la machine lors d'une séance de dialyse avec une pluralité d'étapes d'ultrafiltration en alternance avec des étapes de rétrofiltration est décrite dans la demande internationale WO2013050689A1 ou WO2019150058A2. Cependant, il convient de noter que la phase de dialyse comprend de manière générale une phase d'échange entre le compartiment sang et le compartiment dialysat, par exemple par osmose, et peut ne pas comprendre de phase de rétrofiltration et/ou de phase d'ultrafiltration.

Pour la phase de dialyse, l'unité de pilotage 10 commande en particulier les moyens de mise sous pression des poches 50, 50' et les systèmes à pince C1,C2 ; C1',C2' et C5,C6 ; C5',C6' de fermeture/ouverture du système d'amenée 5 et du système d'évacuation 6. Dans une séance de dialyse, la phase d'amorçage correspond à l'arrivée du sang dans le compartiment sang du dialyseur. Pour cette phase d'amorçage, l'unité de pilotage commande la pompe à sang Psg.

La séance de dialyse se termine par une phase de restitution au cours de laquelle le sang présent dans le circuit sang (ligne veineuse, ligne artérielle, et compartiment sang du patient) est restitué au patient. Pour cette phase de restitution, l'unité de pilotage commande également la pompe à sang Psg, une poche de sérum physiologique étant connectée à la ligne L1 pour permettre de restituer le sang sans risque d'introduction d'air.

Comme détaillé ci-après, l'unité de pilotage 10 est en outre configurée pour mettre en œuvre un procédé de rinçage et d'élimination d'air du circuit extracorporel de la machine de dialyse.

Ladite unité de pilotage 10 se présente sous la forme d'un système électronique et informatique qui comprend par exemple un processeur, tel qu'un microprocesseur, une mémoire de travail et une mémoire de données. Ladite unité de pilotage peut se présenter sous la forme d'un microcontrôleur.

Autrement dit, les fonctions et étapes décrites peuvent être mise en œuvre sous forme de programme informatique ou via des composants matériels (p. ex. des réseaux de portes programmables). En particulier, les fonctions et étapes opérées par l'unité de pilotage ou ses modules peuvent être réalisées par des jeux d'instructions ou modules informatiques enregistrés dans une mémoire pour leur exécution par un processeur ou contrôleur ou être réalisées par des composants électroniques dédiés ou des composants de type FPGA ou ASIC. Il est aussi possible de combiner des parties informatiques et des parties électroniques.

Lorsqu'il est précisé que l'unité ou des moyens ou modules de ladite unité sont configurés pour réaliser une opération donnée, cela signifie que l'unité comprend des instructions informatiques et les moyens d'exécution correspondants qui permettent de réaliser ladite opération et/ou que l'unité comprend des composants électroniques correspondants.

Ladite unité de pilotage présente aussi une interface d'entrée de données. On peut prévoir que ladite interface permette d'entrée des données relatives au patient et/ou aux consommables de la machine.

### • Autres composants ou moyens

Dans l'exemple illustré aux figures, la machine de dialyse est aussi munie de composants classiques pour assurer un traitement fiable et efficace du liquide corporel à traiter, notamment un détecteur de fuite de sang FS ménagé dans la ligne d'évacuation 62. Avantageusement, l'unité de pilotage 10 est configurée pour permettre d'étalonner les débitmètres l'un par rapport à l'autre.

Chaque enceinte 500, 500' est munie de moyens de chauffage ou préchauffage de l'enceinte. Chaque enceinte 500, 500', 600, 600' est aussi munie d'une vanne de mise à l'air V2, V5, V8, V11 associée à un filtre à air F1, F2, F3, F4, et d'un capteur de pression P7, P8, P9 et P10.

Les moyens de mise sous pression 70 comprennent les éléments suivants : un réservoir d'air comprimé R1, un capteur de pression P11 du réservoir R1, un compresseur Pa1, un clapet anti-retour pour l'air comprimé Ar1, et un filtre à air et silencieux pour l'air comprimé Si1.

Le générateur de vide 80 comprend un réservoir de vide d'air R2, un capteur de pression P12 du réservoir R2, une pompe à vide Pa2, un clapet anti-retour pour le vide d'air Ar2 et un filtre à air et silencieux pour le vide d'air Si 2.

La ligne d'amenée 52 est aussi munie de moyens de chauffage Ch3 du dialysat associé à des moyens de mesure de température T1, T2, T3. La ligne d'évacuation 62 est munie de moyens de mesure de température T4, T5 en aval des poches 60, 60'.

La ligne veineuse L2 comprend aussi un organe de fermeture/ouverture CV, tel qu'une pince.

La machine peut comprendre une ligne de by-pass L10, ainsi qu'un ou plusieurs organes Vbp de fermeture/ouverture associés. La ligne de by-pass L10 peut être utilisée pour réaliser l'amorçage et la calibration du circuit de dialysat, et pour permettre de ne pas injecter dans le dialyseur du dialysat défectueux, par exemple du fait d'un problème de température.

Avantageusement, la ligne d'évacuation du dialyseur est raccordée à un récipient de récupération ou à un système de mise à l'égout Egt.

### • Source d'alimentation

La source d'alimentation en dialysat utilisée pour une séance donnée de dialyse est de préférence une ou un ensemble de poches nourricières de plus grand volume, par exemple 5L, que celui, par exemple 150 ml, de ladite poche ventricule 50, 50'.

### • Procédé de rinçage

Un exemple de mise en œuvre d'un procédé de rinçage préalablement à une séance de dialyse est présenté ci-dessous.

Le procédé de rinçage est décrit avec une machine de dialyse telle que présentée ci-avant, mais peut être mis en œuvre avec une autre machine de dialyse.

Une source d'alimentation en dialysat 40 est raccordé au système d'amenée 5 de dialysat. La ligne artérielle L1 est connectée à l'entrée 201 du compartiment sang 12 du dialyseur 100 et la ligne veineuse L2 est connectée à la sortie 202 du compartiment dialysat 14 du dialyseur 100. L'entrée 201 du compartiment sang 12 du dialyseur 100 est située au-dessus de la sortie 202 du compartiment dialysat 14 du dialyseur 100.

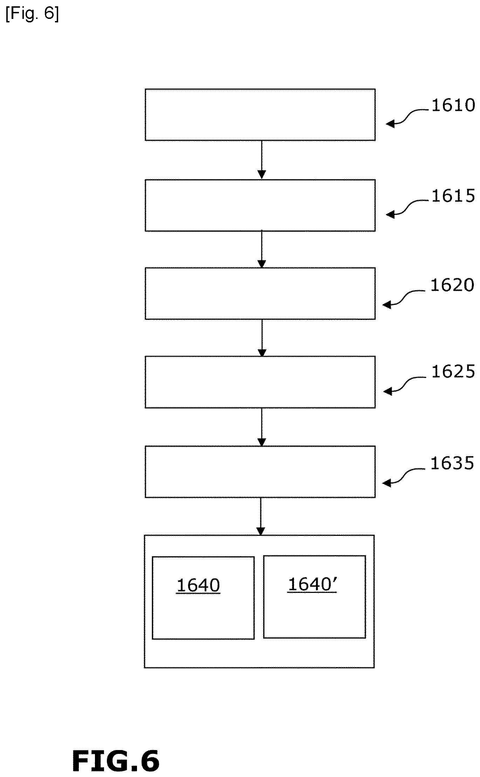

En référence à la Figure 1, à la Figure 4 et aussi à la Figure 6 qui décrit des étapes d'une phase de rinçage, l'opérateur, qui peut être le patient, connecte à l'étape 1610 une poche 1211 de sérum physiologique (stérile) à une extrémité de la ligne artérielle L1 opposée à celle connectée au dialyseur 100.

L'extrémité de la ligne artérielle L1 à laquelle est raccordée la poche 1211 de sérum physiologique peut être une partie en forme de Y comprenant deux branches de raccordement dont une première branche est utilisée pour le raccordement de la ladite poche 1211 et dont la deuxième branche peut être utilisée par la suite pour le raccordement du patient. En particulier, chacune des première et deuxième branches du Y peut être munie d'un raccord rapide pour y raccorder respectivement la poche 1211, puis un dispositif de connexion (par exemple une aiguille artérioveineuse, un cathéter, etc) au patient pour l'accès artériel, et d'un système de pince pour contrôler la fermeture/ouverture de la branche correspondante. Le raccord rapide est par exemple un raccord de type Luer Lock, correspondant à la norme NF EN ISO 594.

A l'étape 1615, l'opérateur connecte à une extrémité de la ligne veineuse L2 opposée à celle connectée au dialyseur 100, une poche 1221 de recueil qui est destinée à collecter le sérum physiologique en provenance de la poche 1211 de sérum physiologique comme détaillé ci-après.

L'extrémité de la ligne veineuse L2, à laquelle est raccordée la poche 1221 de recueil de sérum physiologique, peut ensuite être utilisée pour le raccordement du patient. La ligne veineuse peut être munie d'un raccord rapide, par exemple de type Luer Lock, pour y raccorder respectivement la poche 1221 de recueil, puis un dispositif de connexion (par exemple une aiguille artérioveineuse, un cathéter, etc) au patient pour le retour veineux, et d'un système de pince pour contrôler la fermeture/ouverture de la branche correspondante.

### • Première phase de rinçage

Une première phase de rinçage est réalisée pour faire circuler du sérum physiologique dans le circuit extracorporel. De manière résumée et comme schématisé avec des flèches à la Figure 4, au cours de cette première phase, du sérum physiologique initialement présent dans la poche 1211 de sérum physiologique passe dans la ligne artérielle L1, traverse le compartiment sang 12 du dialyseur, et passe dans la ligne veineuse L2, en entrainant d'éventuels résidus au cours de sa circulation, puis est collecté dans la poche de 1221 de recueil. Le système de pince CV qui permet de fermer ou d'ouvrir la circulation dans la ligne veineuse L2 est ouvert pour permettre au sérum physiologique d'être recueilli dans la poche 1221.

En particulier et en référence à la Figure 1, à la Figure 4 et à la Figure 6, à l'étape 1620, l'unité de pilotage 10 commande le fonctionnement de la pompe à sang Psg dans un sens de rotation donné, de manière à faire circuler le sérum physiologique depuis la poche de sérum physiologique 1211 jusque dans la poche de recueil 1221 à l'intérieur de laquelle il est collecté. La pompe à sang Psg peut être une pompe péristaltique, par exemple une pompe telle que décrite dans la demande WO2013175115A1.

Cette première phase de rinçage correspond à un rinçage du circuit extracorporel qui permet d'évacuer les résidus physiques et/ou chimiques présents dans les composants, correspondant par exemple à des résidus de matière issus de la fabrication des composants du circuit extracorporel.

Avantageusement, pour cette phase de rinçage, l'unité de pilotage 10 commande la pompe Psg de manière à obtenir une variation du débit de sérum physiologique par alternance de 100 mL/min et 350 mL/min.

Selon un aspect particulier, le volume de sérum physiologique utilisé lors de cette première phase est compris entre 600 et 1800 mL.

Selon un mode de réalisation, l'unité de pilotage est configurée pour déterminer le volume de sérum physiologique écoulé de sorte que l'unité de pilotage peut déterminer le moment où le volume de dialysat à utiliser pour la première phase de rinçage est atteint et exécuter une deuxième phase d'élimination d'air présentée ci-après.

Durant cette première phase de rinçage, la circulation dans le circuit de dialysat peut être fermée, du côté du système d'amenée 5 du dialysat et/ou du côté du système d'évacuation 6 de dialysat. En variante, la circulation dans le circuit de dialysat peut être ouverte. En effet, le sérum physiologique s'écoule de la poche 1211 dans la poche 1221 avec une faible pression de sorte que le sérum physiologique traverse le compartiment sang 12 sans passer à travers la membrane 3 dans le compartiment dialysat. Quand bien même du sérum physiologique passerait dans le compartiment dialysat, cela ne serait pas gênant puisque le sérum physiologique, est, comme le dialysat, un liquide stérile et de composition proche de celle du dialysat.

Après cette première phase de rinçage, le système de pince CV est fermé à l'étape 1625. La fermeture du système de pince CV peut être commandée par l'unité de pilotage 10.

Cette circulation du sérum physiologique permet de rincer le circuit extracorporel et ainsi de le nettoyer en enlevant différentes particules ou résidus potentiellement présentes dans le circuit extracorporel qui sont collectées dans la poche de recueil 1221.

Cependant des bulles d'air peuvent s'être accumulées dans la partie haute, référencée ZBA aux Figures 1 et 2, du compartiment sang 12 du dialyseur lors de cette première phase de rinçage.

### • Deuxième phase d'élimination d'air

Ainsi, après la première phase de rinçage présentée ci-avant, une deuxième phase d'élimination d'air est réalisée.

Pour réaliser la deuxième phase d'élimination d'air, on fait circuler du dialysat à travers le dialyseur 100 dans le sens d'une remontée de la ligne artérielle L1 qui est connectée à l'entrée 201 du compartiment sang 12 du dialyseur 100, jusque dans la poche de sérum physiologique 1211 qui a été partiellement vidée au cours de la première phase de rinçage.

Comme rappelé ci-dessus précédemment, la circulation dans la ligne veineuse L2 est fermée.

L'unité de pilotage 10 commande à l'étape 1635 la fermeture de la circulation dans le système d'évacuation du dialysat 6, par exemple au niveau de la ligne d'évacuation 62. A cet effet on peut prévoir que l'unité de pilotage 10 commande le passage en position fermée d'un organe C62 d'ouverture/fermeture présent sur la ligne d'évacuation 62 comme illustré aux Figures 2, 3 et 5. En variante, l'unité de pilotage 10 peut commander tout ou partie des autres organes C5, C5, C5' et/ou C6, C6' de manière à fermer la circulation dans la ligne d'évacuation 62. En particulier, il est possible de simplement fermer C5 et C5'.

Puis, à l'étape 1640, l'unité de pilotage fait fonctionner le système de délivrance 51 de dialysat de sorte que, la circulation à travers la ligne d'évacuation 62 étant fermée, le dialysat qui entre dans le compartiment de dialysat 14 par l'entrée 401 du dialyseur 100, traverse la membrane 3 pour passer dans le compartiment sang 12 et, la ligne veineuse L2 étant fermée, remonte par l'entrée 201 dans la ligne artérielle L1 jusque dans la poche 1211 de sérum physiologique. La circulation de dialysat est schématisée avec des flèches à la Figure 5.

Le dialysat parcourt ainsi la ligne artérielle L1 en sens inverse de ce qui a été réalisé lors de la phase de rinçage avec le sérum physiologique. Le fait de faire circuler le dialysat dans le dialyseur 100 dans le sens d'une remontée de la ligne artérielle L1 permet de chasser l'air présent en partie haute ZBA du dialyseur, c'est-à-dire l'air présent du côté de l'entrée 201 à laquelle est raccordée à la ligne artérielle L1 comme illustré à la Figure 1 et à la Figure 2.

Avantageusement, l'entrée 401 est située en dessous de la sortie 402 de la ligne d'évacuation 62 qui est obturée.

La pompe à sang Psg située du côté du circuit extracorporel est aussi mise en marche, à l'étape 1640', en parallèle du fonctionnement du système de délivrance 51, pour obtenir dans la partie active du circuit extracorporel (parties L1 et 12) un débit de dialysat correspondant sensiblement au débit de dialysat fourni par le système de délivrance 51 de dialysat.

Selon un aspect particulier, lorsque le système de délivrance 51 de dialysat fonctionne, la pompe à sang Psg est commandée de manière à fournir un débit donné (ou débit de consigne) qui est régulé en fonction de la pression mesurée dans la partie active du circuit extracorporel. Ladite pression est mesurée de préférence à l'aide du capteur Pv situé entre le système à pince CV et le dialyseur 100.

Le débit de la pompe à sang Psg est ainsi commandé de sorte que la pression dans la partie active du circuit extracorporel soit comprise dans une plage de valeur de pression prédéfinie, par exemple [0 - 100 mmHg] pour un débit de pompe à sang de 100mL/min ou [0 - 200 mmHg] pour un débit de pompe à sang de 350mL/min.

Lorsque la pression mesurée est supérieure à une valeur seuil alors la pompe à sang Psg est commandée de manière à augmenter le débit dans la ligne artérielle L1 afin de ramener la pression mesurée dans la plage de valeurs prédéfinie et lorsque la pression mesurée est inférieure à une valeur seuil, la pompe à sang Psg est commandée de manière à réduire le débit afin de ramener la pression mesurée à une valeur comprise dans la plage de valeurs prédéfinie.

Selon un aspect particulier, le volume de dialysat utilisé lors de cette deuxième phase d'élimination d'air, par rinçage en sens inverse, est choisi de manière à avoir, à la fin de la phase de rinçage et de la phase d'élimination d'air, un volume donné, par exemple 450mL, restant dans la poche de sérum physiologique pour pouvoir assurer une étape restitution en fin de séance.

Dans la phase de rinçage et/ou d'élimination d'air, on peut prévoir que l'unité de pilotage 10 fasse varier de manière brusque la vitesse de fonctionnement de la pompe Psg afin de générer des à-coups au cours de la circulation et ainsi améliorer encore l'élimination de l'air dans le circuit extracorporel.

Une fois la phase d'élimination d'air achevée, le patient peut ensuite se connecter aux lignes L1 et L2 pour démarrer une séance de dialyse.

En particulier, la poche de recueil 1221 peut être retirée de la ligne veineuse L2 en ouvrant le connecteur Luer Lock et le dispositif de connexion du patient peut être connecté sur cette ligne veineuse L2 précédemment équipée de la poche de recueil 1221.

Pendant les deux étapes du processus de rinçage, le système de pince présent sur la branche du système en Y équipée de la poche de sérum physiologique est ouvert et celui présent sur l'autre branche du système en Y est fermée.

Avant le démarrage d'une séance de dialyse, le système de pince présent sur la branche du système en Y reliée à la poche de sérum physiologique est fermé.

En fin de séance de dialyse, le système de pince de la branche du système en Y qui est raccordée au patient est fermé, et le système de pince de la branche du système en Y qui est raccordée à la poche de sérum physiologique est ouvert pour assurer la restitution du sang.

Comme rappelé ci-dessus, lorsque cela est techniquement admissible, l'ordre de certaines étapes peut être inversé et le procédé de rinçage et d'élimination d'air peut comprendre entre deux étapes décrites d'autres étapes supplémentaires.

L'invention n'est pas limitée aux modes de réalisation illustrés dans les dessins. De plus, le terme «comprenant» n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Machine de dialyse permettant de traiter un liquide corporel, tel que du sang ou du plasma, ladite machine comportant :
- un dialyseur (100) comprenant une enceinte qui inclut :
- une première zone de passage (12) de liquide, appelée compartiment sang, qui présente une entrée (201), dite entrée de liquide corporel, et une sortie (202), dite sortie de liquide corporel, et
- une deuxième zone de passage (14) de dialysat, appelée compartiment dialysat, qui présente une entrée (401) de dialysat et une sortie (402) de dialysat ;
- un système de membrane (3) entre la première zone de passage (12) de liquide et la zone de passage (14) de dialysat ;
- une ligne artérielle (L1) raccordée à l'entrée (201) de liquide corporel du compartiment sang (12) du dialyseur (100) ; la ligne artérielle (L1) présentant une extrémité à laquelle un premier contenant (1211), telle qu'une poche, de sérum physiologique est raccordable ;
- une ligne veineuse (L2) raccordée à la sortie (202) de liquide corporel du compartiment sang (12) du dialyseur (100) ; la ligne veineuse (L2) présentant une extrémité à laquelle un deuxième contenant (1221), telle qu'une poche, est raccordable ;
- une pompe (Psg), appelée pompe à sang, configurée pour permettre de faire circuler un liquide dans la ligne artérielle (L1) dans un sens et dans l'autre ;
- un système d'amenée du dialysat (5) comprenant :
- une ligne d'amenée (52) de dialysat qui est raccordée à l'entrée (401) de la zone de passage de dialysat (14), et
- un système de délivrance (51) de dialysat raccordé à la ligne d'amenée (52), et auquel est raccordable une source d'alimentation (40) en dialysat ;
- un système d'évacuation du dialysat (6) comprenant une ligne d'évacuation (62) de dialysat qui est raccordée à la sortie (402) du compartiment de dialysat (14) ;
l'unité de pilotage (10) étant configurée pour, à l'état raccordé, du premier contenant (1211) à la ligne artérielle (L1), du deuxième contenant (1221) à la ligne veineuse (L2), et de la source d'alimentation (40) en dialysat au système de délivrance (51) de dialysat,
mettre en œuvre une première phase de rinçage, appelée phase de rinçage de circuit extracorporel, qui comprend une étape de commande (1620) de fonctionnement de la pompe à sang (Psg) de manière à faire circuler le sérum physiologique contenu dans le premier contenant (1211) jusque dans le deuxième contenant (1221) ;
**caractérisée en ce que** l'unité de pilotage (10) est configurée pour mettre en œuvre une phase d'élimination d'air dans le dialyseur (100), qui comprend les étapes suivantes:
- commander la fermeture (1625) de circulation dans la ligne veineuse (L2) ;
- commander la fermeture (1635) de circulation dans la ligne d'évacuation (62) du système d'évacuation du dialysat (6);
- commander le fonctionnement (1640) du système de délivrance (51) du dialysat pour faire circuler du dialysat depuis la source d'alimentation (40) en dialysat à travers le compartiment sang (12) et la ligne artérielle (L1).

2. Machine selon la revendication 1, dans laquelle l'entrée (201) de liquide corporel du dialyseur (100) est située au-dessus de la sortie (202) de liquide corporel.

3. Machine selon la revendication 1 ou 2, dans laquelle la ligne veineuse (L2) est munie d'un système de pince (CV), commandable par l'unité de pilotage (10), pour permettre de fermer ou d'ouvrir la circulation à l'intérieur de la ligne veineuse (L2).

4. Machine selon l'une quelconque des revendications précédentes, dans laquelle, pour l'exécution de la phase d'élimination d'air, l'unité de pilotage (10) est configurée pour commander le fonctionnement de la pompe à sang (Psg), dans le sens inverse du sens de fonctionnement utilisé lors de la phase de rinçage, parallèlement à ladite étape de commande de fonctionnement du système de délivrance (51) du dialysat.

5. Machine selon l'une quelconque des revendications précédentes, dans laquelle la machine de dialyse comprenant un capteur de pression (Pv) agencé pour mesurer la pression dans le circuit de circulation de dialysat formé entre la pompe (Psg) et le système de délivrance (51) de dialysat, l'unité de pilotage (10) est configurée pour piloter la vitesse de fonctionnement de la pompe (Psg) en fonction de la pression mesurée de manière à maintenir ladite pression dans une plage de valeur prédéfinie.

6. Machine selon l'une quelconque des revendications précédentes, dans laquelle l'unité de pilotage (10) est configurée pour, dans la phase de rinçage et/ou d'élimination d'air, faire varier la vitesse de fonctionnement de la pompe (Psg) afin de générer des à-coups.

7. Machine selon l'une quelconque des revendications précédentes, dans laquelle ledit système de délivrance (51) comprend au moins une poche (50) souple, appelée poche ventricule, destinée à contenir du dialysat, et des moyens de mise sous pression de la poche ventricule (50).

8. Machine selon l'une quelconque des revendications précédentes, **caractérisée en ce que** l'unité de pilotage (10) est configurée pour déterminer le volume de sérum physiologique déplacé par la pompe à sang (Psg) jusque dans le deuxième contenant (1221), et pour arrêter de faire fonctionner ladite pompe à sang (Psg) lorsque le volume de sérum physiologique déplacé a atteint une valeur prédéfinie, par exemple comprise entre 600 et 1800 mL.

9. Procédé de rinçage et d'élimination d'air pour une machine de dialyse, ladite machine de dialyse comportant :
- un dialyseur (100) comprenant une enceinte qui inclut :
- une première zone de passage (12) de liquide appelée compartiment sang, qui présente une entrée (201), dite entrée de liquide corporel, et une sortie (202), dite sortie de liquide corporel, et
- une deuxième zone de passage (14) de dialysat, appelée compartiment dialysat, qui présente une entrée (401) de dialysat et une sortie (402) de dialysat ;
- un système de membrane (3) entre la première zone de passage (12) de liquide et la zone de passage (14) de dialysat ;
- une ligne artérielle (L1) raccordée à l'entrée (201) de liquide corporel du compartiment sang (12) du dialyseur (100) ; la ligne artérielle (L1) présentant une extrémité à laquelle un premier contenant (1211), telle qu'une poche, de sérum physiologique est raccordable ;
- une ligne veineuse (L2) raccordée à la sortie (202) de liquide corporel du compartiment sang (12) du dialyseur (100) ; la ligne veineuse (L2) présentant une extrémité à laquelle un deuxième contenant (1221), telle qu'une poche, est raccordable ;
- une pompe (Psg), appelée pompe à sang, configurée pour permettre de faire circuler un liquide dans la ligne artérielle (L1) dans un sens et dans l'autre ;
- un système d'amenée du dialysat (5) comprenant :
- une ligne d'amenée (52) de dialysat qui est raccordée à l'entrée (401) de la zone de passage de dialysat (14), et
- un système de délivrance (51) de dialysat raccordé à la ligne d'amenée (52), et auquel est raccordable une source d'alimentation (40) en dialysat ;
- un système d'évacuation du dialysat (6) comprenant une ligne d'évacuation (62) de dialysat qui est raccordée à la sortie (402) du compartiment de dialysat (14),
ledit procédé comprenant une première phase de rinçage, appelée phase de rinçage de circuit extracorporel, qui comprend une étape de commande (1620) de fonctionnement de la pompe à sang (Psg) de manière à faire circuler le sérum physiologique contenu dans le premier contenant (1211) jusque dans le deuxième contenant (1221),
**caractérisé en ce que** ledit procédé comprend aussi une phase d'élimination d'air dans le dialyseur (100), qui comprend les étapes suivantes :
- fermeture (1625) de circulation dans la ligne veineuse (L2) ;
- fermeture (1635) de circulation dans la ligne d'évacuation (62) du système d'évacuation du dialysat (6) ;
- fonctionnement (1640) du système de délivrance (51) du dialysat pour faire circuler du dialysat depuis la source d'alimentation (40) en dialysat à travers le compartiment sang (12) et la ligne artérielle (L1).

10. Procédé selon la revendication 9, dans lequel la phase d'élimination d'air comprend le fonctionnement (1640') de la pompe à sang (Psg), dans le sens inverse du sens de fonctionnement utilisé lors de la phase de rinçage, parallèlement à ladite étape de fonctionnement du système de délivrance (51) du dialysat.

11. Produit de programme informatique non transitoire comprenant des instructions de code de programme pour l'exécution des étapes d'un procédé conforme à l'une quelconque des revendications 9 ou 10, lorsque ledit programme est exécuté par un processeur d'une unité de pilotage (10) d'une machine de dialyse selon l'une quelconque des revendications 1 à 8.

## Patentansprüche

1. Dialysemaschine zur Behandlung einer Körperflüssigkeit, wie Blut oder Plasma, wobei die Maschine Folgendes aufweist:
- ein Dialysegerät (100) mit einem Gehäuse, das Folgendes aufweist:
- einen ersten Durchflussbereich (12) für Flüssigkeit, der als Blutkammer bezeichnet wird und einen Einlass (201), der als Körperflüssigkeitseinlass bezeichnet wird, und einen Auslass (202), der als Körperflüssigkeitsauslass bezeichnet wird, und
- einen zweiten Durchflussbereich (14) für Dialysat, der als Dialysatkammer bezeichnet wird und einen Dialysateinlass (401) und einen Dialysatauslass (402) aufweist;
- ein Membransystem (3) zwischen dem ersten Durchflussbereich (12) für Flüssigkeit und dem Durchflussbereich (14) für Dialysat;
- eine Arterienleitung (L1), die mit dem Körperflüssigkeitseinlass (201) der Blutkammer (12) des Dialysegeräts (100) verbunden ist; wobei die Arterienleitung (L1) ein Ende aufweist, an das ein erster Behälter (1211), beispielsweise ein Beutel, mit physiologischer Kochsalzlösung angeschlossen werden kann;
- eine Venenleitung (L2), die mit dem Körperflüssigkeitsauslass (202) der Blutkammer (12) des Dialysegeräts (100) verbunden ist; wobei die Venenleitung (L2) ein Ende aufweist, an das ein zweiter Behälter (1221), beispielsweise ein Beutel, angeschlossen werden kann;
- eine Pumpe (Psg), die als Blutpumpe bezeichnet wird und so konfiguriert ist, dass sie eine Flüssigkeit in der Arterienleitung (L1) in beide Richtungen zirkulieren lässt;
- ein Dialysat-Zufuhrsystem (5), das Folgendes aufweist:
- eine Dialysat-Zuleitung (52), die mit dem Einlass (401) des Durchflussbereichs (14) für Dialysat verbunden ist, und
- ein Dialysat-Abgabesystem (51), das mit der Zuleitung (52) verbunden ist und an das eine Dialysat-Versorgungsquelle (40) angeschlossen werden kann;
- ein Dialysat-Ableitungssystem (6), das eine Dialysat-Ableitungsleitung (62) aufweist, die mit dem Auslass (402) der Dialysatkammer (14) verbunden ist;
wobei die Steuereinheit (10) so konfiguriert ist, dass sie im angeschlossenen Zustand den ersten Behälter (1211) mit der Arterienleitung (L1), den zweiten Behälter (1221) mit der Venenleitung (L2) und die Dialysat-Versorgungsquelle (40) mit dem Dialysat-Abgabesystem (51) verbindet,
eine erste Spülphase durchführt, die als Spülphase des extrakorporalen Kreislaufs bezeichnet wird und einen Schritt (1620) zum Steuern des Betriebs der Blutpumpe (Psg) aufweist, um die in dem ersten Behälter (1211) enthaltene physiologische Kochsalzlösung in den zweiten Behälter (1221) zu zirkulieren;
**dadurch gekennzeichnet, dass** die Steuereinheit (10) so konfiguriert ist, dass sie eine Entlüftungsphase im Dialysegerät (100) durchführt, die die folgenden Schritte aufweist:
- Steuern der Schließung (1625) des Durchflusses in der Venenleitung (L2);
- Steuern der Schließung (1635) des Durchflusses in der Ableitungsleitung (62) des Dialysat-Ableitungssystems (6);
- Steuern des Betriebs (1640) des Dialysat-Abgabesystems (51), um Dialysat aus der Dialysat-Versorgungsquelle (40) durch die Blutkammer (12) und die Arterienleitung (L1) zirkulieren zu lassen.

2. Maschine nach Anspruch 1, wobei der Körperflüssigkeitseinlass (201) des Dialysegeräts (100) oberhalb des Körperflüssigkeitsauslasses (202) angeordnet ist.

3. Maschine nach Anspruch 1 oder 2, wobei die Venenleitung (L2) mit einem von der Steuereinheit (10) steuerbaren Klemmsystem (CV) versehen ist, um den Durchfluss innerhalb der Venenleitung (L2) zu schließen oder zu öffnen.

4. Maschine nach einem der vorhergehenden Ansprüche, wobei zur Durchführung der Entlüftungsphase die Steuereinheit (10) so konfiguriert ist, dass sie den Betrieb der Blutpumpe (Psg) in umgekehrter Richtung zu der während der Spülphase verwendeten Betriebsrichtung parallel zu dem Schritt des Steuerns des Betriebs des Dialysat-Abgabesystems (51) steuert.

5. Maschine nach einem der vorhergehenden Ansprüche, wobei die Dialysemaschine einen Drucksensor (Pv) aufweist, der so angeordnet ist, dass er den Druck im Dialysat-Zirkulationskreislauf misst, der zwischen der Pumpe (Psg) und dem Dialysat-Abgabesystem (51) gebildet wird, wobei die Steuereinheit (10) so konfiguriert ist, dass sie die Betriebsgeschwindigkeit der Pumpe (Psg) in Abhängigkeit vom gemessenen Druck so steuert, dass der Druck in einem vordefinierten Wertebereich gehalten wird.

6. Maschine nach einem der vorhergehenden Ansprüche, wobei die Steuereinheit (10) so konfiguriert ist, dass sie in der Spül- und/oder Entlüftungsphase die Betriebsgeschwindigkeit der Pumpe (Psg) variiert, um Stöße zu erzeugen.

7. Maschine nach einem der vorhergehenden Ansprüche, wobei das Abgabesystem (51) mindestens einen flexiblen Beutel (50), einen sogenannten Ventrikelbeutel, zur Aufnahme von Dialysat und Mittel zum Unterdrucksetzen des Ventrikelbeutels (50) aufweist.

8. Maschine nach einem der vorhergehenden Ansprüche, **dadurch gekennzeichnet, dass** die Steuereinheit (10) so konfiguriert ist, dass sie das von der Blutpumpe (Psg) in den zweiten Behälter (1221) verdrängte Volumen an physiologischer Kochsalzlösung bestimmt und den Betrieb der Blutpumpe (Psg) beendet, wenn das verdrängte Volumen an physiologischer Kochsalzlösung einen vordefinierten Wert erreicht hat, beispielsweise zwischen 600 und 1800 ml.

9. Verfahren zum Spülen und Entlüften einer Dialysemaschine, wobei die Dialysemaschine Folgendes aufweist:
- ein Dialysegerät (100) mit einem Gehäuse, das Folgendes aufweist:
- einen ersten Durchflussbereich (12) für Flüssigkeit, der als Blutkammer bezeichnet wird, und einen Einlass (201), der als Körperflüssigkeitseinlass bezeichnet wird, und einen Auslass (202), der als Körperflüssigkeitsauslass bezeichnet wird, und
- einen zweiten Durchflussbereich (14) für Dialysat, der als Dialysatkammer bezeichnet wird und einen Dialysateinlass (401) und einen Dialysatauslass (402) aufweist;
- ein Membransystem (3) zwischen dem ersten Durchflussbereich (12) für Flüssigkeit und dem Durchflussbereich (14) für Dialysat;
- eine Arterienleitung (L1), die mit dem Körperflüssigkeitseinlass (201) der Blutkammer (12) des Dialysegeräts (100) verbunden ist; wobei die Arterienleitung (L1) ein Ende aufweist, an das ein erster Behälter (1211), beispielsweise ein Beutel, mit physiologischer Kochsalzlösung angeschlossen werden kann;
- eine Venenleitung (L2), die mit dem Körperflüssigkeitsauslass (202) der Blutkammer (12) des Dialysegeräts (100) verbunden ist; wobei die Venenleitung (L2) ein Ende aufweist, an das ein zweiter Behälter (1221), beispielsweise ein Beutel, angeschlossen werden kann;
- eine Pumpe (Psg), die als Blutpumpe bezeichnet wird und so konfiguriert ist, dass sie eine Flüssigkeit in der Arterienleitung (L1) in beide Richtungen zirkulieren lässt;
- ein Dialysat-Zufuhrsystem (5), das Folgendes aufweist:
- eine Dialysat-Zuleitung (52), die mit dem Einlass (401) des Durchflussbereichs (14) für Dialysat verbunden ist, und
- ein Dialysat-Abgabesystem (51), das mit der Zuleitung (52) verbunden ist und an das eine Dialysat-Versorgungsquelle (40) angeschlossen werden kann;
- ein Dialysat-Ableitungssystem (6), das eine Dialysat-Ableitungsleitung (62) aufweist, die mit dem Auslass (402) der Dialysatkammer (14) verbunden ist,
wobei das Verfahren eine erste Spülphase aufweist, die als extrakorporale Kreislaufspülphase bezeichnet wird und einen Schritt (1620) zum Steuern des Betriebs der Blutpumpe (Psg) aufweist, um die in dem ersten Behälter (1211) enthaltene physiologische Kochsalzlösung in den zweiten Behälter (1221) zu zirkulieren;
**dadurch gekennzeichnet, dass** das Verfahren auch eine Entlüftungsphase im Dialysegerät (100) aufweist, die die folgenden Schritte aufweist:
- Schließen (1625) des Durchflusses in der Venenleitung (L2);
- Schließen (1635) des Durchflusses in der Ableitungsleitung (62) des Dialysat-Ableitungssystems (6);
- Betreiben (1640) des Dialysat-Abgabesystems (51), um Dialysat aus der Dialysat-Versorgungsquelle (40) durch die Blutkammer (12) und die Arterienleitung (L1) zirkulieren zu lassen.

10. Verfahren nach Anspruch 9, wobei die Entlüftungsphase den Betrieb (1640') der Blutpumpe (Psg) in umgekehrter Richtung zu der während der Spülphase verwendeten Betriebsrichtung parallel zu dem genannten Betriebsschritt des Dialysat-Abgabesystems (51) aufweist.

11. Nichtflüchtiges Computerprogrammprodukt, das Programmcodeanweisungen zum Ausführen der Schritte eines Verfahrens gemäß einem der Ansprüche 9 oder 10 aufweist, wenn das Programm von einem Prozessor einer Steuereinheit (10) einer Dialysemaschine gemäß einem der Ansprüche 1 bis 8 ausgeführt wird.

## Claims

1. A dialysis machine for treating a bodily fluid, such as blood or plasma, said machine having:
- a dialyzer (100) comprising an enclosure which includes:
- a first passage zone (12) for liquid, called a blood compartment, which has an inlet (201), called the bodily fluid inlet, and an outlet (202), called the bodily fluid outlet, and
- a second passage zone (14) for dialysate, called a dialysate compartment, which has a dialysate inlet (401) and a dialysate outlet (402);
- a membrane system (3) between the first passage zone (12) for liquid and the passage zone (14) for dialysate;
- an arterial line (L1) connected to the bodily fluid inlet (201) of the blood compartment (12) of the dialyzer (100); the arterial line (L1) having an end to which a first container (1211), such as a bag, of physiological saline is connectable;
- a venous line (L2) connected to the bodily fluid outlet (202) of the blood compartment (12) of the dialyzer (100); the venous line (L2) having an end to which a second container (1221), such as a bag, is connectable;
- a pump (Psg), called a blood pump, configured to allow a fluid to circulate back and forth in the arterial line (L1);
- a dialysate feed system (5) comprising:
- a dialysate feed line (52) which is connected to the inlet (401) of the dialysate passage zone (14), and
- a dialysate delivery system (51) which is connected to the feed line (52) and to which a dialysate supply source (40) is connectable;
- a dialysate discharge system (6) comprising a dialysate discharge line (62) which is connected to the outlet (402) of the dialysate compartment (14);
the control unit (10) being configured such that, in the connected state of the first container (1211) to the arterial line (L1), of the second container (1221) to the venous line (L2), and of the dialysate supply source (40) to the dialysate delivery system (51), it implements a first rinsing phase, called an extracorporeal circuit rinsing phase, which comprises a step of control (1620) of the operation of the blood pump (Psg) so as to cause the physiological saline, contained in the first container (1211), to circulate into the second container (1221);
**characterized in that** the control unit (10) is configured to implement a phase of air elimination from the dialyzer (100), which comprises the following steps:
- controlling the closure (1625) of circulation in the venous line (L2);
- controlling the closure (1635) of circulation in the discharge line (62) of the dialysate discharge system (6);
- controlling the operation (1640) of the dialysate delivery system (51) in order to cause dialysate to circulate from the dialysate supply source (40) through the blood compartment (12) and the arterial line (L1).

2. The machine as claimed in claim 1, in which the bodily fluid inlet (201) of the dialyzer (100) is situated above the bodily fluid outlet (202).

3. The machine as claimed in claim 1 or 2, in which the venous line (L2) is provided with a clamp system (CV), controllable by the control unit (10), to permit closing or opening of the circulation inside the venous line (L2).

4. The machine as claimed in any one of the preceding claims, in which, for execution of the air elimination phase, the control unit (10) is configured to control the operation of the blood pump (Psg), in the opposite direction to the direction of operation used during the rinsing phase, in parallel with said step of controlling the operation of the dialysate delivery system (51).

5. The machine as claimed in any one of the preceding claims, in which, with the dialysis machine comprising a pressure sensor (Pv) arranged to measure the pressure in the dialysate circulation circuit formed between the pump (Psg) and the dialysate delivery system (51), the control unit (10) is configured to control the speed of operation of the pump (Psg) as a function of the measured pressure so as to maintain said pressure within a predefined value range.

6. The machine as claimed in any one of the preceding claims, in which the control unit (10) is configured such that, in the phase of rinsing and/or elimination of air, it varies the speed of operation of the pump (Psg) in order to generate jolts.

7. The machine as claimed in any one of the preceding claims, in which said delivery system (51) comprises at least one flexible bag (50), called a ventricle bag, intended to contain dialysate, and means for pressurizing the ventricle bag (50).

8. The machine as claimed in any one of the preceding claims, **characterized in that** the control unit (10) is configured to determine the volume of physiological saline displaced by the blood pump (Psg) into the second container (1221), and to stop operation of said blood pump (Psg) when the volume of physiological saline displaced has reached a predefined value, for example between 600 and 1800 ml.

9. A method of rinsing and eliminating air from a dialysis machine, said dialysis machine having:
- a dialyzer (100) comprising an enclosure which includes:
- a first passage zone (12) for liquid, called a blood compartment, which has an inlet (201), called the bodily fluid inlet, and an outlet (202), called the bodily fluid outlet, and
- a second passage zone (14) for dialysate, called a dialysate compartment, which has a dialysate inlet (401) and a dialysate outlet (402);
- a membrane system (3) between the first passage zone (12) for liquid and the passage zone (14) for dialysate;
- an arterial line (L1) connected to the bodily fluid inlet (201) of the blood compartment (12) of the dialyzer (100); the arterial line (L1) having an end to which a first container (1211), such as a bag, of physiological saline is connectable;
- a venous line (L2) connected to the bodily fluid outlet (202) of the blood compartment (12) of the dialyzer (100); the venous line (L2) having an end to which a second container (1221), such as a bag, is connectable;
- a pump (Psg), called a blood pump, configured to allow a fluid to circulate back and forth in the arterial line (L1);
- a dialysate feed system (5) comprising:
- a dialysate feed line (52) which is connected to the inlet (401) of the dialysate passage zone (14), and
- a dialysate delivery system (51) which is connected to the feed line (52) and to which a dialysate supply source (40) is connectable;
- a dialysate discharge system (6) comprising a dialysate discharge line (62) which is connected to the outlet (402) of the dialysate compartment (14), said method comprising a first rinsing phase, called an extracorporeal circuit rinsing phase, which comprises a step of control (1620) of the operation of the blood pump (Psg) so as to cause the physiological saline, contained in the first container (1211), to circulate into the second container (1221),
**characterized in that** said method also comprises a phase of air elimination from the dialyzer (100), which comprises the following steps:
- closure (1625) of circulation in the venous line (L2);
- closure (1635) of circulation in the discharge line (62) of the dialysate discharge system (6);
- operation (1640) of the dialysate delivery system (51) in order to cause dialysate to circulate from the dialysate supply source (40) through the blood compartment (12) and the arterial line (L1).

10. The method as claimed in claim 9, in which the phase of air elimination comprises operation (1640') of the blood pump (Psg), in the opposite direction to the direction of operation used during the rinsing phase, in parallel with said step of operating the dialysate delivery system (51).

11. A non-transient computer program product comprising program code instructions for executing the steps of a method as claimed in either of claims 9 and 10, when said program is executed by a processor of a control unit (10) of a dialysis machine as claimed in any one of claims 1 to 8.
